(19) Europäisches Patentamt
European Patent Office
Office européen des brevets

(11) Veröffentlichungsnummer: **0 150 765**
**B1**

# (12) EUROPÄISCHE PATENTSCHRIFT

(45) Veröffentlichungstag der Patentschrift: **22.03.89**

(51) Int. Cl.⁴: **A 61 K 7/13**, C 07 C 127/15

(21) Anmeldenummer: **85100398.8**

(22) Anmeldetag: **16.01.85**

## (54) Verwendung von p-Ureidoalkylamino-nitrobenzolverbindungen in Haarfärbemitteln und neue p-Ureidoalkylamino-nitrobenzolverbindungen.

(30) Priorität: **26.01.84 DE 3402519**

(43) Veröffentlichungstag der Anmeldung: **07.08.85 Patentblatt 85/32**

(45) Bekanntmachung des Hinweises auf die Patenterteilung: **22.03.89 Patentblatt 89/12**

(84) Benannte Vertragsstaaten: **AT BE CH DE FR GB IT LI LU NL SE**

(56) Entgegenhaltungen:
**DE-A- 1 945 451**
**GB-A- 1 150 445**

(73) Patentinhaber: **Wella Aktiengesellschaft, Berliner Allee 65, D-6100 Darmstadt (DE)**

(72) Erfinder: **Clausen, Thomas. Dr., Rheinstrasse 19, D-6108 Weiterstadt (DE)**
Erfinder: **Konrad, Eugen, Mecklenburger Strasse 101, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Gegenstand der Erfindung ist die Verwendung von Nitrofarbstoffen zum Färben von Haaren, wobei als Nitrofarbstoffe p-Ureidoalkylamino-nitrobenzolverbindungen verwendet werden, sowie neue p-Ureidoalkylamino-nitrobenzolverbindungen.

Nitrofarbstoffe finden heute in Haarfärbemitteln eine breite Anwendung. Sie werden in Oxidationshaarfärbemitteln als Zusätze für die Erzeugung von natürlichen oder modischen Farbnuancen verwendet. Durch Kombination von mehreren verschieden gefärbten Nitrofarbstoffen ist es jedoch auch möglich, Haarfärbemittel herzustellen, die Haare in natürlichen bis modischen Nuancen ohne die Anwendung von Oxidationsmitteln zu färben vermögen.

So können zum Beispiel durch die Kombination eines orangefärbenden mit einem blaufärbenden Nitrofarbstoff natürlich wirkende braune Färbungen erzeugt werden. Daneben ist es jedoch auch möglich, mit einem gelbfärbenden und einem violettfärbenden Nitrofarbstoff ein ähnliches Ergebnis zu erhalten.

Es werden daher besonders Nitrofarbstoffe benötigt, die das Haar in einem intensiven reinen Zitronengelb einzufärben vermögen; welches möglichst frei von Rotanteilen sein muss. Daneben sind noch viele weitere Anforderungen gestellt. Die Nitrofarbstoffe müssen in toxikologischer und dermatologischer Hinsicht unbedenklich sein. Ihre Verwendung in Oxidationshaarfärbemitteln setzt voraus, dass sie in Anwesenheit von Wasserstoffperoxid in alkalischer Lösung stabil sind. Ausserdem sollten sie gegenüber Reduktionsmitteln, die häufiger Bestandteil von Oxidationshaarfärbemitteln sind, stabil sein. Weiterhin wird für die erzeugten Haarfärbungen eine gute Licht-, Säure- und Reibeechtheit gefordert. Schliesslich sollten die Nitroverbindungen durch möglichst einfache und preisgünstige Verfahren darstellbar sein.

Die bisher in der Literatur zur Gelbfärbung des Haares beschriebenen substituierten Amino-nitrophenole erfüllen die vorstehend genannten Voraussetzungen nur unzureichend. Das im International Journal of Cosmetic Science 1982, Seiten 25–35 genannte 4-Nitro-3-(2′-hydroxyethylamino)-phenol ergibt zwar eine zitronengelbe Färbung, diese ist aber sehr farbschwach. Zwei weitere Isomere, nämlich das 4-Nitro- und das 5-Nitro-2-(2′-hydroxyethylamino)-phenol, sind keine gelbfärbenden, sondern orangefärbende Nitrofarbstoffe mit störendem Rotanteil (siehe DE-B 928 909 und DE-A 3 231 455). Der zuletzt genannte Nitrofarbstoff ist überdies sehr pH-empfindlich und zeigt unerwünschte Farbänderungen bei Säure- bzw. Alkalieinwirkung.

Weitere bekannte gelbe Nitrofarbstoffe sind die in der DE-B 1 619 395 genannten o- und p-Nitroanilinderivate. Diese Verbindungen erfüllen zwar weitgehend die Anforderungen in anwendungstechnischer Hinsicht, befriedigen aber nicht im Hinblick auf die physiologischen Eigenschaften.

Es wurde nun überraschenderweise gefunden, dass sich die genannten Nachteile beseitigen lassen durch die Verwendung einer 1-Ureidoalkylamino-4-nitrobenzolverbindung der allgemeinen Formel I

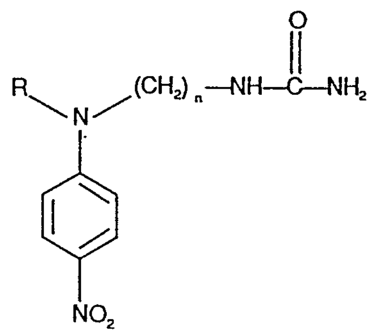

(I),

worin R Wasserstoff oder einen Hydroxyalkylrest mit 2 bis 4 Kohlenstoffatomen darstellt und n eine ganze Zahl von 2 bis 4 bedeutet, als Farbstoff in Haarfärbemitteln.

Die Feststellung, dass Verbindungen der allgemeinen Formel I alle an gelbfärbende Nitrofarbstoffe für Haarfärbemittel gestellten Anforderungen erfüllen, ist umso überraschender, weil eine Verbindung der allgemeinen Formel I mit R = H und n = 2 schon seit längerer Zeit aus der DE-A 1 945 451 als Vorstufe für die Synthese der als Farbstoffbase für Oxidationshaarfärbemittel empfohlenen Verbindung N-(β-Ureidoethyl)-paraphenylendiamin bekannt ist.

Von den für die erfindungsgemässe Verwendung beschriebenen Farbstoffen der Formel I sind solche, in denen R einen Hydroxyalkylrest mit 2 bis 4 Kohlenstoffatomen bedeutet, neu.

Gegenstand der vorliegenden Erfindung sind daher auch neue 1-[N-(Hydroxyalkyl), N-(ureidoalkyl)-amino]-4-nitrobenzole der allgemeinen Formel II

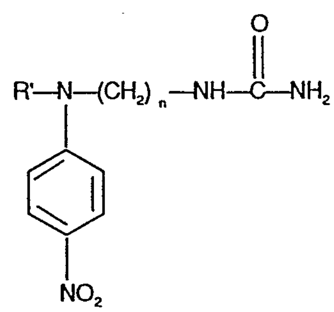

(II),

wobei R′ einen Hydroxyalkylrest mit 2 bis 4 Kohlenstoffatomen bedeutet und n eine ganze Zahl von 2 bis 4 darstellt.

Diese Verbindungen zeichnen sich gegenüber solchen der Formel I mit R = H durch eine bessere Wasserlöslichkeit aus.

Die p-Ureidoalkylamino-nitrobenzole der Formel I (welche die neuen Verbindungen der Formel II einschliessen) können aus preiswerten Vorstufen wie folgt hergestellt werden:

Ausgehend von p-Chlor- oder p-Fluornitrobenzol III erfolgt, gemäss nachstehendem Reaktionsschema, ein nucleophiler Austausch des Halogens mit einem Alkylendiamin IV, wobei in guter Ausbeute das Aminoalkylnitroanilin V erhalten wird. Dieses wird sodann mit einem Alkalicyanat in saurer Lösung in das gewünschte p-Ureidoalkylamino-nitrobenzol der Formel I übergeführt.

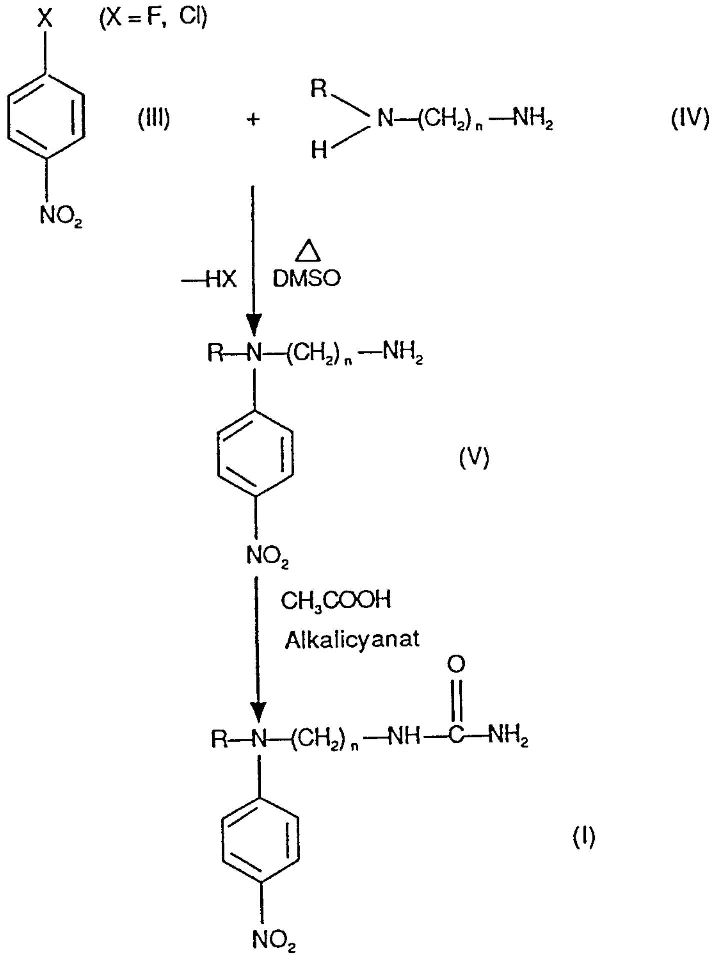


Die als Zwischenprodukt benötigten Aminoalkylnitroaniline V lassen sich auch vorteilhaft durch die Gabriel-Reaktion herstellen, indem, wie in US-A 3 332 948 erwähnt, p-Nitroanilin VI mit den Phthalimidderivaten von Alkylenhalogeniden VII umgesetzt wird

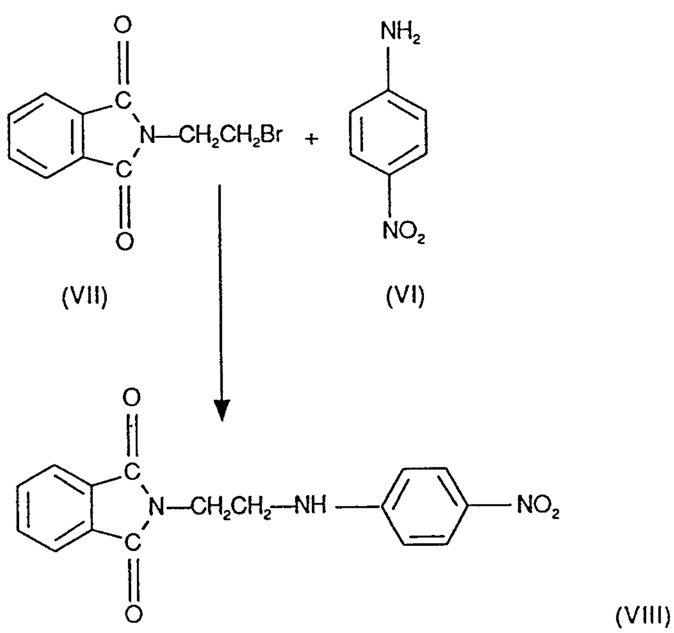

und anschliessend das Amin aus dem Phthalimid VIII freigesetzt wird.

Überraschend sind die sehr guten toxikologischen und physiologischen Eigenschaften der Verbindungen nach der allgemeinen Formel I. So sind zum Beispiel die beiden Farbstoffe 1-[(2'-Ureidoethyl)-amino]-4-nitrobenzol und 1-[N(2'-Ureidoethyl), N(2''-hydroxyethyl)-amino]-4-nitrobenzol im Amestest nicht bzw. nur schwach mutagen und weisen eine ausgesprochen geringe akute Toxizität auf.

Von besonderem Vorteil sind weiterhin die ausserordentlich hohe Farbtiefe des mit den Verbindungen der Formel I erhältlichen rein zitronengelben Farbtons und die Beständigkeit gegenüber Basen wie Ammoniak und gegenüber Reduktionsmitteln wie zum Beispiel Ascorbinsäure, wodurch auch eine Verwendung in Oxidationshaarfärbemitteln möglich ist.

Die vorliegende Erfindung betrifft ebenfalls Mittel zur Färbung von Haaren mit einem Farbstoffgehalt und für Haarfärbemittel üblichen Zusätzen, dadurch gekennzeichnet, dass es eine p-Ureidoalkylamino-nitrobenzolverbindung der Formel I enthält.

Die erfindungsgemässen Mittel zur Färbung von Haaren betreffen sowohl solche, die ohne Zugabe eines Oxidationsmittels angewendet werden, als auch solche, bei denen die Zugabe eines Oxidationsmittels erforderlich ist.

Bei den ersteren Haarfärbemitteln ohne Oxidationsmittel-Zugabe handelt es sich um diejenigen, die neben den Farbstoffen der angegebenen Formel noch andere direkt auf das Haar aufziehende Farbstoffe enthalten können. Von diesen für die Haarfärbung bekannten Farbstoffen seien beispielsweise die folgenden Klassen erwähnt:

Aromatische Nitrofarbstoffe (zum Beispiel 1,4-Diamino-2-nitrobenzol und dessen Derivate), Azofarbstoffe (zum Beispiel Acid Brown 4, C.I. 14805), Anthrachinonfarbstoffe (zum Beispiel Disperse Violet 4, C.I. 61105), Triphenylmethanfarbstoffe (zum Beispiel Basic Violet 1, C.I. 42535), wobei die Farbstoffe dieser Klassen je nach der Art ihrer Substituenten sauren, nichtionogenen oder basischen Charakter haben können. Weitere geeignete direkt auf das Haar aufziehende Farbstoffe sind beispielsweise in dem Buch von J.C. Johnson, «Hair Dyes» Noyes Data Corp. Park-Ridge (USA) (1973), Seiten 3 - 91 und 113 - 139 (ISBN:0-8155-0477-2) beschrieben.

Mit diesen erfindungsgemässen Haarfärbemitteln, die Verbindungen der Formel I im Gemisch mit den vorstehend genannten Farbstoffen enthalten, lassen sich neben reinen Modetönen auch modische Blond- und Brauntöne von hervorragender Stabilität erzielen.

Die Zubereitungsform der hier beschriebenen Haarfärbemittel auf der Basis von direkt auf das Haar aufziehenden Farbstoffen kann beispielsweise eine Lösung, insbesondere eine wässrige oder wässrig-alkoholische Lösung, sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Die Farbstoffe der angegebenen Formel I sollen in diesen Färbemitteln ohne Oxidationsmittel-Zugabe in einer Konzentration von 0,01 bis 1,0 Gew.%, vorzugsweise von 0,01 bis 0,5 Gew.%, enthalten sein. Der Gesamtgehalt an den direkt ziehenden Farbstoffen liegt in den Grenzen von 0,01 bis 3,0 Gew.%. Der pH-Wert dieser Färbemittel liegt im Bereich von 3 bis 10,5, insbesondere bei pH 7,5 bis 9,5, wobei die Einstellung des gewünschten alkalischen pH-Wertes hauptsächlich mit Ammoniak erfolgt, jedoch auch mit organischen Aminen wie beispielsweise Monoethanolamin oder Triethanolamin vorgenommen werden kann.

Ihre Anwendung erfolgt in üblicher Weise durch Aufbringen einer für die Haarfärbung ausreichenden Menge des Mittels auf die Haare, mit denen es eine Zeitlang, 5 bis 30 Minuten, in Berührung bleibt. Anschliessend wird mit Wasser, gegebenenfalls noch mit einer wässrigen Lösung einer schwachen organischen Säure, gespült und getrocknet. Als schwache organische Säure können beispielsweise Essigsäure, Zitronensäure, Weinsäure und ähnliche Verwendung finden.

Die vorstehend beschriebenen Haarfärbemittel ohne Oxidationsmittel-Zugabe können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Acrylsäure- bzw. Methacrylsäurepolymerisate, basische Polymerisate von Estern aus diesen beiden Säuren und Aminoalkoholen bzw. deren Salze oder Quaternisierungsprodukte, Polyacrylnitril, Polyvinyllactame sowie Copolymerisate aus derartigen Verbindungen wie Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von 1 bis 5 Gew.% enthalten. Die pH-Werte der Mittel liegen im Bereich von 6,0 bis 9,0.

Die Anwendung dieser Haarfärbemittel mit zusätzlicher Festigung erfolgt in bekannter und üblicher Weise durch Befeuchten des Haares mit dem Festiger, Festlegen (Einlegen) des Haares zur Frisur und anschliessende Trocknung.

Selbstverständlich können die vorstehend beschriebenen Haarfärbemittel ohne Oxidationsmittel-Zugabe gegebenenfalls weitere für Haarfärbemittel übliche Zusätze wie zum Beispiel Pflegestoffe, Netzmittel, Verdicker, Weichmacher und Parfümöle sowie auch andere, nachstehend für Oxidationshaarfärbemittel aufgeführte übliche Zusätze enthalten.

Zum Gegenstand der vorliegenden Erfindung

gehören auch, wie eingangs erwähnt, diejenigen Haarfärbemittel, bei denen die Zugabe eines Oxidationsmittels erforderlich ist. Sie enthalten ausser den Farbstoffen gemäss der angegebenen Formel I und gegebenenfalls bekannten direkt auf das Haar aufziehenden Farbstoffen noch zusätzlich bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole wie beispielsweise p-Toluylendiamin, p-Phenylendiamin, p-Aminophenol und ähnliche Verbindungen, welche zum Zwecke der Nuancierung der Färbungen mit sogenannten Modifiern, wie zum Beispiel m-Phenylendiamin, Resorcin, m-Aminophenol und anderen kombiniert werden.

Derartige zur Haarfärbung bekannte und übliche Oxidationsfarbstoffe sind unter anderem in dem Buch von E. Sagarin, «Cosmetics», Science and Technology (1957), Interscience Publishers Inc., New York, Seiten 503 ff. sowie in dem Buch von H. Janistyn, «Handbuch der Kosmetika und Riechstoffe» (1973) Seiten 338 ff., beschrieben.

Mit Mischungen aus diesen Oxidationsfarbstoffen und den Farbstoffen gemäss der angegebenen Formel I lassen sich neben reinen Modetönen auch modische Blond- und Brauntöne erhalten.

Die Farbstoffe gemäss der Formel I sind in diesen Färbemitteln mit Oxidationsmittel-Zugabe in einer Konzentration von 0,01 bis 1,0 Gew.%, vorzugsweise 0,05 bis 0,5 Gew.%, enthalten. Der Gesamtgehalt an Farbstoffen in diesen Färbemitteln beträgt 0,1 bis 5,0 Gew.%.

Oxidationshaarfärbemittel sind im allgemeinen alkalisch eingestellt, vorzugsweise auf pH-Werte von 8,0 bis 11,5, wobei die Einstellung insbesondere mit Ammoniak erfolgt. Es können dazu aber auch andere organische Amine, zum Beispiel Monoethanolamin oder Triethanolamin, Verwendung finden. Als Oxidationsmittel zur Entwicklung der Haarfärbungen kommen hauptsächlich Hydrogenperoxyd und dessen Additionsverbindungen in Betracht. Die Zubereitungsform dieser Haarfärbemittel kann die gleiche wie bei Haarfärbemitteln ohne Oxidationsmittel-Zugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate wie Carboxymethylcellulose, Alginate, Vaseline, Paraffinöl und Fettsäuren sowie ausserdem Pflegestoffe wie Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von 0,5 bis 30 Gew. %, während die Verdicker in einer Menge von 0,1 bis 25 Gew. % in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen 50 bis 150 ml, auf das Haar aufträgt. Nach einer für die Haarfärbung ausreichenden Einwirkungszeit, welche üblicherweise 10 bis 45 Minuten beträgt, wird mit Wasser, gegebenenfalls anschliessend mit der wässrigen Lösung einer schwachen organischen Säure, wie zum Beispiel Zitronensäure oder Weinsäure, gespült und getrocknet.

Hinsichtlich der färberischen Möglichkeiten bieten die erfindungsgemässen Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die nachstehenden Beispiele sollen den Anmeldungsgegenstand erläutern.

Beispiele für Mittel zur Färbung des Haares

| Beispiel 1 | Flüssiges Haarfärbemittel |
|---|---|
| 0,10 g | 1-[(2'-Ureidoethyl)-amino]-4-nitrobenzol |
| 0,50 g | Hydroxyethylcellulose |
| 5,00 g | Laurylalkohol-diglykolethersulfat-Natriumsalz, 28%ige wässrige Lösung |
| 15,00 g | Isopropylalkohol |
| 0,03 g | Ammoniak, 25%ig |
| 79,37 g | Wasser |
| 100,00 g | |

30 ml des flüssigen Haarfärbemittels der obigen Zusammensetzung werden auf weisse menschliche Haare aufgetragen und 10 Minuten einwirken gelassen. Nach der Spülung mit Wasser und dem Trocknen ist das Haar leuchtendgelb gefärbt.

| Beispiel 2 | Farbfestiger |
|---|---|
| 0,05 g | 1-[(2'-Ureidoethyl)-amino]-4-nitrobenzol |
| 2,00 g | Polyvinylpyrrolidon |
| 0,10 g | Glycerin |
| 40,00 g | Isopropylalkohol |
| 57,85 g | Wasser |
| 100,00 g | |

Weisse menschliche Haare werden mit 20 ml der vorstehenden festigenden Färbelösung zur Frisur eingelegt und getrocknet. Das Haar ist leuchtendgelb gefärbt und gefestigt.

| Beispiel 3 | Oxidationshaarfärbemittel |
|---|---|
| 0,05 g | 1-[N-(2'-Hydroxyethyl),N-(2''-ureidoethyl)-amino]-4-nitrobenzol |
| 35,00 g | Ölsäure |
| 15,00 g | Isopropylalkohol |
| 18,00 g | Ammoniak, 25%ig |
| 0,20 g | Dinatriumsalz der Ethylendiamin-tetraessigsäure |
| 0,30 g | Ascorbinsäure |
| 0,40 g | p-Toluylendiamin-sulfat |
| 0,15 g | Resorcin |
| 0,03 g | m-Aminophenol |
| 30,87 g | Wasser |
| 100,00 g | |

50 ml des vorstehenden Haarfärbemittels werden kurz vor dem Gebrauch mit 50 ml Hydrogenperoxydlösung (6%ig) vermischt. Das entstandene Gel wird anschliessend auf graue menschliche Haare aufgetragen und 30 Minuten einwirken gelassen. Sodann wird mit Wasser gespült und getrocknet. Das Haar hat einen natürlichen Blondton erhalten.

| Beispiel 4 | Oxidationshaarfärbemittel in Cremeform |
|---|---|
| 0,10 g | 1-[(2'-Ureidoethyl)-amino]-4-nitrobenzol |
| 1,50 g | p-Phenylendiamin |
| 0,50 g | m-Aminophenol |
| 1,00 g | 3,5-Diamino-2,6-dimethoxypyridin-dihydrochlorid |
| 0,30 g | Ascorbinsäure |
| 15,00 g | Cetylalkohol |
| 3,50 g | Laurylalkohol-diglykolethersulfat-Natriumsalz (28%ige wässrige Lösung) |
| 6,00 g | Ammoniak, 25%ig |
| 72,10 g | Wasser |
| 100,00 g | |

50 ml des vorstehenden Haarfärbemittels werden kurz vor der Anwendung mit 50 ml Wasserstoffperoxydlösung (6%ig) gemischt. Das Gemisch wird anschliessend auf graue menschliche Haare aufgetragen und 30 Minuten bei einer Temperatur von 40°C einwirken gelassen. Nach dem Spülen des Haares mit Wasser und anschliessendem Trocknen ist dieses ohne Farbstich tiefschwarz eingefärbt.

Herstellungsbeispiel
Stufe 1
1-[N-(2'-Aminoethyl),
N-(2''-hydroxyethyl)-amino]-4-nitrobenzol

15,7 g (0,1 Mol) 4-Nitrochlorbenzol und 30 g N-(2-Hydroxyethyl)-ethylendiamin werden in 20 ml Dimethylsulfoxid gelöst und 4 Stunden lang auf 140°C erwärmt. Anschliessend wird das Reaktionsgemisch auf Eis gegossen und das unlösliche Reaktionsprodukt abfiltriert. Nach dem Trocknen wird aus Essigester umkristallisiert. Man erhält 12,0 g (53% der Theorie) der reinen Verbindung in Form gelber Kristalle, die einen Schmelzpunkt von 126°C aufweisen.

Stufe 2
Herstellung von
1-[N-2'-Hydroxyethyl),N-(2''-ureidoethyl)-amino]-4-nitrobenzol

12,0 g des Nitrobenzolderivates aus Stufe 1 werden in 20 ml Eisessig unter Erwärmung gelöst. Nach dem Entfernen des Heizbades werden 5,2 g Kalimcyanat portionsweise zugegeben. Anschliessend erwärmt man noch 15 Minuten lang und verdünnt sodann mit Wasser. Die gewünschte Ureidoverbindung fällt aus. Sie wird abfiltriert und aus Wasser umkristallisiert. Man erhält 4,0 g (28% der Theorie) der reinen Ureidoverbindung vom Schmelzpunkt 196°C.

Protonen-Kernresonanzspektrum (NMR):
Angaben in δ (ppm) Standard: Tetramethylsilan
Lösungsmittel: hexadeuteriertes Dimethylsulfoxid ($DMSO-d_6$)

| | |
|---|---|
| 8,00 | (Dublett, 3-H, 5-H, J = 9,5 Hz) |
| 7,32 | (breit, NH) |
| 6,68 | (Dublett, 2-H, 6-H, J = 9,5 Hz) |
| 5,88 | (Singulett, scharf, $NH_2$) |
| 4,84 | (Triplett, OH, J = 5 Hz) |
| 3,60-3,15 | (Multiplett $CH_2$) |

**Patentansprüche**

1. Verwendung einer 1-Ureidoalkylamino-4-nitrobenzolverbindung der allgemeinen Formel I

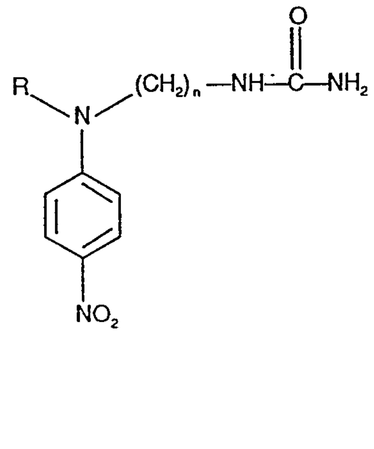

(I),

wobei R Wasserstoff oder einen Hydroxyalkylrest mit 2 bis 4 Kohlenstoffatomen darstellt und n eine ganze Zahl von 2 bis 4 bedeutet, als Farbstoff in Haarfärbemitteln.

2. Mittel zur Färbung von Haaren mit einem Farbstoffgehalt und für Haarfärbemittel üblichen Zusätzen, dadurch gekennzeichnet, dass es 0,01 bis 1,0 Gew.% einer 1-Ureidoalkylamino-4-nitrobenzolverbindung der Formel I enthält.

3. Mittel nach Anspruch 2, dadurch gekennzeichnet, dass es 0,01 bis 1,0 Gew.% 1-[(2'-Ureidoethyl)-amino]-4-nitrobenzol enthält.

4. Mittel nach Anspruch 2, zusätzlich enthaltend mindestens einen bekannten Oxidationshaarfarbstoff.

5. Mittel nach Anspruch 4, dadurch gekennzeichnet, dass es einen pH-Wert von 8,0 bis 11,5 aufweist.

6. Mittel in Form eines Haarfärbemittels mit zusätzlicher Haarfestigung nach den Ansprüchen 2 und 3, zusätzlich enthaltend in wässrig-alkoholischer Lösung bekannte direkt auf das Haar aufziehende Farbstoffe und mindestens ein kosmetisches Polymerisat.

7. 1-[N-(Hydroxyalkyl), N-(ureidoalkyl)-amino]-4-nitrobenzol der allgemeinen Formel II

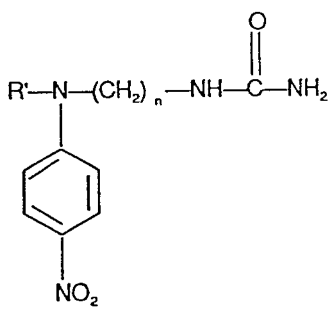

(II),

wobei R' einen Hydroxyalkylrest mit 2 bis 4 Kohlenstoffatomen bedeutet und n eine ganze Zahl von 2 bis 4 darstellt.

8. 1-[N-(2'-Hydroxyethyl), N-(2''-ureidoethyl)-amino]-4-nitrobenzol.

**Claims**

1. Use of a 1-ureidoalkylamino-4-nitrobenzene compound to the general formula I

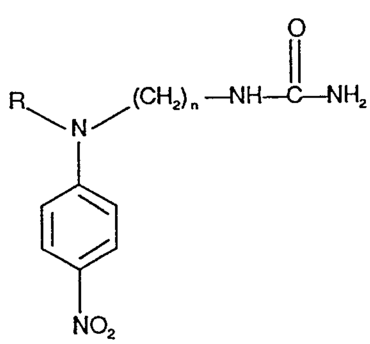

(I),

in which R represents hydrogen or a hydroxyalkyl radical with 2 to 4 carbon atoms and in which n is a whole number of 2 to 4, as a dyestuff in hair colourants.

2. Composition for colouring hair with a dyestuffs content and with additives normally used with hair colouring agents, characterised in that it contains 0.01 to 1.0% by weight of a 1-ureidoalkylamino-4-nitrobenzene compound to formula I.

3. Composition according to Claim 2, characterised in that it contains 0.01 to 1.0% by weight of a 1-[(2'-ureidoethyl)-amino]-4-nitrobenzene.

4. Composition according to Claim 2, additionally containing at least one prior art oxidation hair colourant.

5. Composition according to Claim 4, characterised in that it has a pH value of 8.0 to 11.5.

6. Composition in the form of a hair colourant with an additional hair setting effect according to Claims 2 and 3, also containing in aqueous-alcohol solution known direct dyeing dyestuffs and at least one cosmetic polymerisate.

7. 1-[N-(hydroxyalkyl), N-(ureidoalkyl)-amino]-4-nitrobenzene to the general formula II

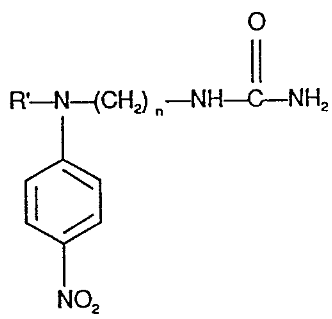

(II)

in which R' is a hydroxyalkyl radical with 2 to 4 carbon atoms and n is a whole number from 2 to 4.

8. 1-[N-(2'-hydroxyethyl), N-(2"-ureidoethyl)-amino]-4-nitrobenzene.

**Revendications**

1. Utilisation d'un composé 1-uréidoalkylamino-4-nitrobenzène de formule générale I:

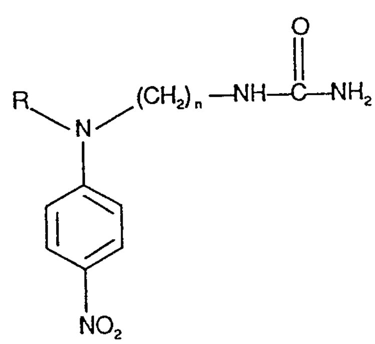

(I),

ou R represente un atom d'hydrogène ou un radical hydroxyalkyle avec 2 à 4 atomes de carbone et n est un nombre entier de 2 à 4, en tant que colorant dans un composition de teinture des cheveux.

2. Composition pour teindre les cheveux avec une teneur en colorant et des adjuvants habituels pour les compositions de teinture des cheveux, caractérisé en ce qu'il contient de 0,01 à 1,0% en poids d'un composé 1-uréidoalkylamino-4-nitrobenzène de la formule I.

3. Composition selon la revendication 2, caractérisé en ce qu'il contient de 0,01 à 1,0% en poids de 1-[(2'-uréidoéthyle)-amino]-4-nitrobenzène.

4. Composition selon la revendication 2, contenant en plus au moins un colorant des cheveux par oxydation connu.

5. Composition selon la revendication 4, caractérisé en ce qu'il présente une valeur de pH de 8,0 à 11,5.

6. Composition sous la forme d'un agent de teinture des cheveux avec fixation additionelle des cheveux selon les revendications 2 et 3, contenant en plus, dans une solution alcoolisée aqueuse, des colorants directs connus à appliquer sur les cheveux et au moins un polymérisat cosmétique.

7. 1-[N-(hydroxyalkyle),N-(uréidoalkyle)-amino]-4-nitrobenzène de formule générale II:

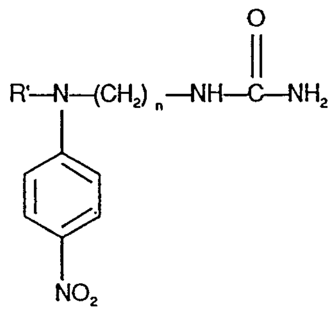

(II)

ou R représente un radical hydroxyalkyle avec 2 à 4 atomes de carbone et n est un nombre entier de 2 a 4.

8. 1-[N-(2'-hydroxyéthyle),N-(2"-uréidoéthyle)-amino]-4-nitrobenzène.